Europäisches Patentamt

European Patent Office

Office européen des brevets

(11) Publication number: **0 505 811 A2**

## EUROPEAN PATENT APPLICATION

(21) Application number: **92103997.0**

(22) Date of filing: **09.03.92**

(51) Int. Cl.⁵: **C12N 15/12**, C12N 15/70, A61K 37/02

(30) Priority: **26.03.91 ES 9100800**

(43) Date of publication of application:
**30.09.92 Bulletin 92/40**

(84) Designated Contracting States:
**AT BE CH DE DK ES FR GB GR IT LI LU NL PT SE**

(71) Applicant: **BOEHRINGER INGELHEIM INTERNATIONAL G.M.B.H.**

**W-6507 Ingelheim am Rhein(DE)**

(72) Inventor: **Giménez Gallego, Guillermo, Prof. Dr.**
**Alberto Aquilera, 23**
**E-28015 Madrid(ES)**
Inventor: **Cuevas Sanchez, Pedro, Dr.**
**Dr. Guiu, 18**
**E-28034 Madrid(ES)**

(54) **Improved acidic fibroblast growth factor (aFGF) and its preparation.**

(57) The present invention relates to a process for obtaining a new plasmide for producing the acidic fibroblast growth factor (aFGF) in its integral form, comprising adding to any codifying nucleotiede sequence of the complete aminoacid sequence of the aFGF the necessary sequences for binding the mRNA to the ribosome and termination of translation, obtaining an intermediate plasmide, and inclusion of the fragment which comprises the aFGF gen and the ribosome binding and termination nucleotide sequence in a vector after potent promotors / operators and a highly expressed protein separated by in phase termination codons..

The fibroblast growth factor has application as drug in protecting injuries produced in the myocardium by ischemia/reperfusion.

EP 0 505 811 A2

The invention refers to a process for obtaining a new plasmide for producing an improved acidic fibroblast growth factor (aFGF) in its integral form by genetic engineering techniques in a microorganism, preferably a procaryotic microorganism, most preferably Escherichia coli, purified without utilizing reverse phase chromotography, as well as using the factor for protection against myocardium ischemia / reperfusion injuries.

The integral form of the aFGF is about three times more active than other forms of this factor in which part of the aminoacids of the amino - terminal end have been removed, being the only ones it has, thus far, been possible to synthesize in E. coli by genetic recombinant techniques; furthermore, the production of aFGF using reverse phase chromatography, produces a modified form of the protein.

Two are the microorganisms that can be chosen to produce, on an industrial scale, a protein by genetic recombinant techniques, namely E. coli and the yeast Saccharomyces cerevisiae. At the present time E. coli displays advantages over the yeast, due to the following properties:

- Ease in its genetical manipulation and stability of the transformed strains;
- Wide variety of vectors of a large numer of copies, with powerful expression promotors which are available and, therefore, high yield of the synthesis of the product desired in relation to the total mass of the culture; and
- Ease and low cost of the growth of this microorganism.

In the case of the aFGF, the expression in yeasts encounters the additional drawback that the human protein has two potential glycosylation sites (Giménez-Gallego et al. 1986, Biochem. Biophys. Res. Commun. 138, 611-617). However, the protein, as isolated from the human brain, does not show this modification. Hence, the risk is run that, on synthesizing the aFGF in an organism in which this protein is foreign, it may end up being glycosylated. Pharmacological use of a glycosylated form of the human aFGF, especially if this modification is performed on an organism so distant in the evolutionary scale as Saccharomyces cerevisiae, involves very high risks. Accordingly, production of the protein in an organism such as E. coli, in which this glycosylation cannot take place, should be considered as a safety factor not to be discounted. From this, a mammalian aFGF in its unglycosylated form is a preferred embodiment of the invention.

Pursuant to the available data in the literature, it is not possible to produce, in E. coli, the integral form of the protein following the guidelines of Spanish Patent 2001967, (Jaye, M. et al. 1987, J. Biol. Chem. 262, 16612-16617), because of the high stabilization energies of the secondary structures that form at the 5'end of the FGF mRNA (Knoerzer, W. et al. 1989, Gene 75 (1), 21-30).

The shortened forms that have been produced to date have the additional modification, compared to the native form, of having the aminoacid methionine in the amino - terminal end (Jaye, M. et al. 1987, J. Biol. Chem. 262, 16612-16617; Linemeyer, D.L. et al. 1987, Bio/technology 5, 960-965).

The pharmacological use of this shortened form can give rise to anaphylactic problems.

In the cases taken up in the quoted literature, aFGF is purified from E. coli cultures by a process which includes reverse phase chromatography (Jaye, M. et al. and Linemayer, D.L. et al., ibidem.). The protein purified by this procedure is nonsoluble in aqueous solutions at neutral or physiological pH, specially at temperatures near that of warm - blooded organisms, impeding its clinical application. On the other hand, since, in accordance with the process for purifying the aFGF the brain, the molecule has to be water soluble (Thomas, K.A. et al. 1984. Proc. Natl. Acad. Sci. USA 81, 357-361), the reverse phase chromatography necessarily should introduce structural modifiactions into the protein and, therefore, affect its physiological properties. On the other hand, by modifying the structure of the molecule new epitopes may appear, giving rise to the possibility of undesirable anaphylactic reactions from the standpoint of clinical application of the aFGF.

Patent document WO 89/00198 in the name of Biotechnology Research Associates, J.V., filed 6 July 1988, deals with the synthesis and manipulation of analogues of the fibroblast growth factor useful for accelerating the healing of wounds, bone fractures, burns, damaged myocardical tissue, degenerated neurological tissue or other traumas. In said document there are also taken up recombinant DNA sequences, which codify acidic and basic FGF analogues, such as recombinant vectors bearing these DNA sequences.

The process object of the invention allows to produce, in a procaryotic microorganism, preferably in a bacterium, most preferably in E. coli, the full form of aFGF without methionine at the amino - terminal end and in wholly soluble form in aqueous solutions. This form is more active than the shorter forms.

According to the invention, the process for obtaining a new plasmid for producing aFGF in its integral form by genetic engineering techniques comprises the following steps:

1. Adding, at the beginning and end, repectively, the necessary sequences for binding the mRNA to the ribosome and for effective termination of the transcription to any nucleotide sequence codifying the complete aFGF aminoacid sequence.

2. Inclusion of the fragment synthesized in 1. in an expression vector.

In accordance with the invention in its preferred embodiment, the aFGF gen is located behind the amino-terminal end of the E. coli lipoprotein, separated therefrom by two codons of phase termination, and set under the control of the lipoprotein promoter and operator - promoter.

The acidic fibroblast growth factor itself, using the pasmid obtained by the process described above, is likewise an object of the present invention. This aFGF is of greater activity than the known sequences, lacks methionine at the amino - terminal end, has not post - translational modifications and is soluble in aqueous solutions at physiological pH by purification without reverse phase chromatography.

Yet another object of the invention is the use of the fibroblast growth factor as drug for protecting the myocardium against ischemic / reperfusion injuries.

The invention is next described, with the aid of the accompanying drawings. They show:

- Figure 1, the map of the plasmid for expression of the complete form of aFGF; INA N-TER = hybrid lipoprotein N-terminal, Pro = promotor, Po = polypeptide
- Figure 2, the result of the electrophoresis under denaturalizing conditions, evidencing that the eluted protein corresponds to a single polypeptide;
- Figures 3a to 3c, respective graphs showing insolubility measurements of the aFGF, prepared by reverse phase and the solubility of the factor without this reverse phase; CIA = absorbency

    3a:    FA = reverse phase, AC = aqueous, without reverse phase

    3b:    reverse phase, pH 7

    3c:    avoidance of precipitation in the presence of heparine, pH 7, 37°C, INA = heparine, DTT = dithiotreitol

- Figure 4, a graph representing the activity of the long form aFGF with respect to shorter forms aFGF;

$\boxed{\text{[}\!\rule[0.4ex]{2em}{0.4pt}\!\text{]}}$ = long form,    $\lozenge\!\rule[0.4ex]{2em}{0.4pt}\!\lozenge$ = short form,

with regard to Ac and Ao see text;
and

- Figures 5a to 5e, respective graphs showing the effect of the FGF, both acid and basic, applied before, simultaneous and after the reperfusion.

    5a:    bFGF injected on reperfusing, I = heart without infarction, II = heart with infarction, III = infarction + FGF

    5b:    bFGF 5 min. prior to reperfusing

    5c:    aFGF 1 min. after reperfusing

    5d:    bFGF 1 min. after reperfusing

    5e:    aFGF no mitogen 1 min. after reperfusing

The invention will next be described in greater detail, with the aid of some examples, it being understood that same are merely illustrative and in no way limitative.

EXAMPLES

SYNTHESIS OF THE GEN WHICH CODIFIES THE COMPLETE FORM OF THE aFGF

In this example, one started from the plasmides PDH 14 (ATCC 53 335) and PDH 15 (ATCC 53 336). Hence, by means of site directed mutagenesis the Nco I restriction site, existing at the height of the 242 nucleotide of the gen, in the fragment included in the PDH 15 plasmide, between the restriction sites EcoR I and Hind III, was eliminated, the plasmide named D12 being obtained. Next, the plasmides PDH 14 and D 12 were fused, using the restriction site Mst II and, at the beginning and end respectively, the necessary sequences were added for joining the mRNA to the ribosome and an effective termination of the transcription, giving rise to a new plasmide named D 18. Then the fragment included between the sites EcoR I and Hind III of the plasmide D 18, which includes the aFGF gen plus the ribosome binding and termination sequence, was included between the same restriction sites in the pIN-IIIA3 vector (Masui, Y. et al. Experimental Manipulation of Gene Expression, Chapter 2, 1283, Academic Press), giving rise to the plasmide D 60.

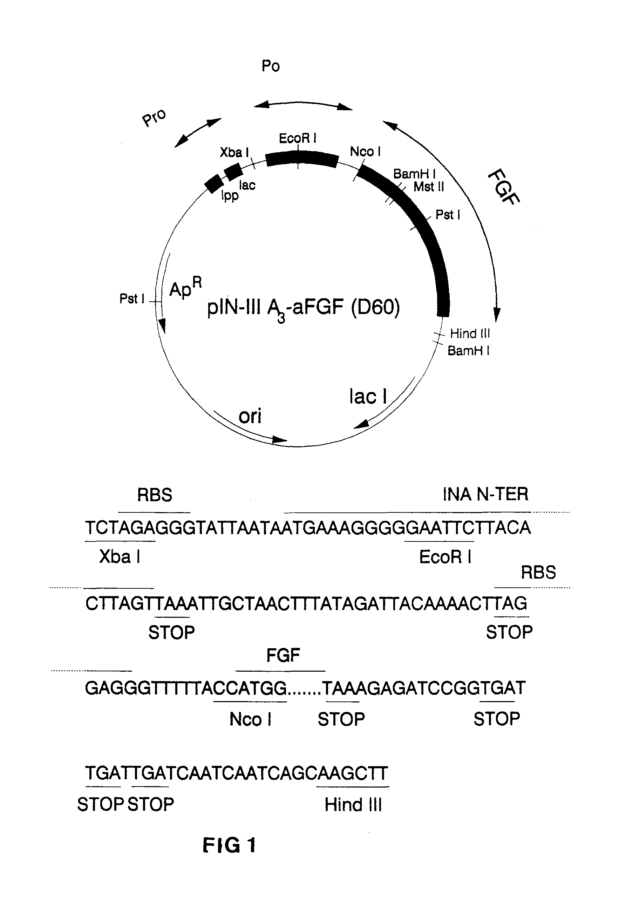

As can be observed from Figure 1, in this construction the aFGF gen is located behind the amino - terminal moiety of the E. coli lipoprotein and separated therefrom by two termination codons in phase, under the control of the powerful promoter of the lipoprotein and of the promoter - operator lacUV5.

SYNTHESIS OF THE ACIDIC FIBROBLAST GROWTH FACTOR (aFGF)

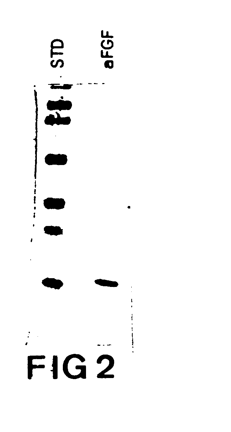

For the aFGF synthesis Lon⁻, Lac⁺ strains of E. coli were used, transformed with the plasmide D60. The cells were allowed to grow in a rich medium with 1,6% lactose until an optical density at 600 nm of approximately 7, being collected by centrifugation and kept frozen at - 20° C until future processing. For the purification of the aFGF, the pellet of frozen cells was suspended at 4° C in a sodium phosphate buffer solution 10 mM pH 7.2 to which tetra - acetate ethylendinitryl was added up to 5 mM, phenylmethyl - sulfonyl fluoride up to 40 ug/ml, L-1-chlorine-3-(4-tosylamide)-4-phenyl-2-butanone up to 100 ug/ml, pepstatine up to 0.7 ug/ml, leupeptine up to 0.5 ug/ml and aprotinine up to 0.5 ug/ml. The cells suspension was passed once through a French press, collecting the cellular lysate directly on heparine - sepharose balanced in the same buffer solution. This mixture was collected in a wide pore sintered glass funnel where it was abundantly washed with the same buffer solution to which NaCl has been added up to 0.3 M. The heparine - sepharose, resuspended in this same buffer solution with NaCl, was then charged into a chromatography column through which the same buffer solution in which the NaCl concentration has been raised to 0.8 M is passed, until the optical absorption at around 280 nm of the eluent is approximately the same as that of the buffer used for the elution. Next, the aFGF was eluted by means of the same buffer solution to which NaCl has been added up to 1.5 M. The protein eluted with this solution corresponds to a single polypeptide, as shown by electrophoresis under denaturalizing conditions (Figure 2).

DIRECT SEQUENCING

The direct sequencing of the purified aFGF was carried out in an Applied Biosystem 477 automatic protein sequencer provided with an on line detector of phenyltiohydantoinamino acids. The obtained sequence shows that the first four aminoacids at the amino - terminus of the protein are alanine, glutamic acid, glycine, glutamic acid. This sequence exactly corresponds to the one codified by the complete aFGF gen.

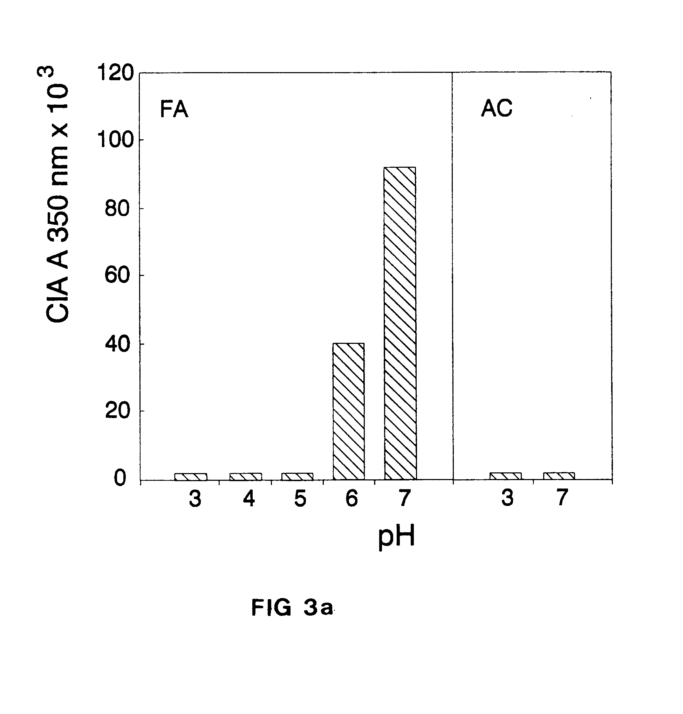

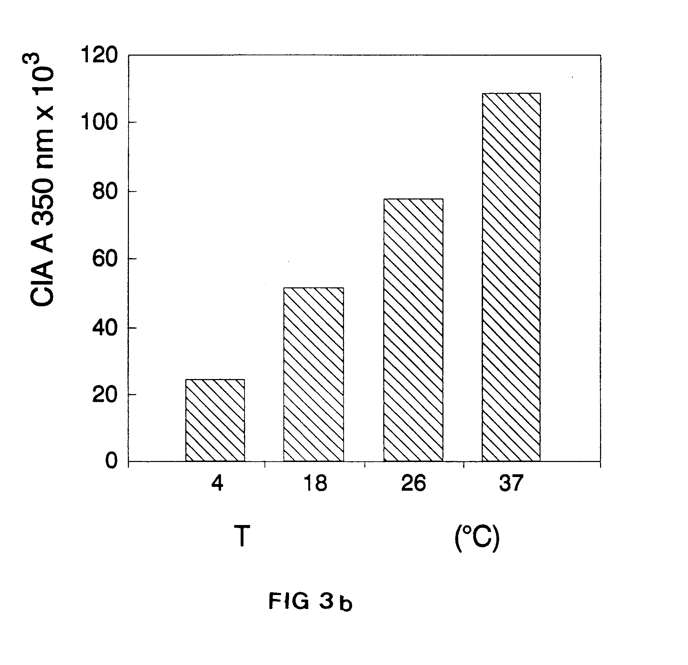

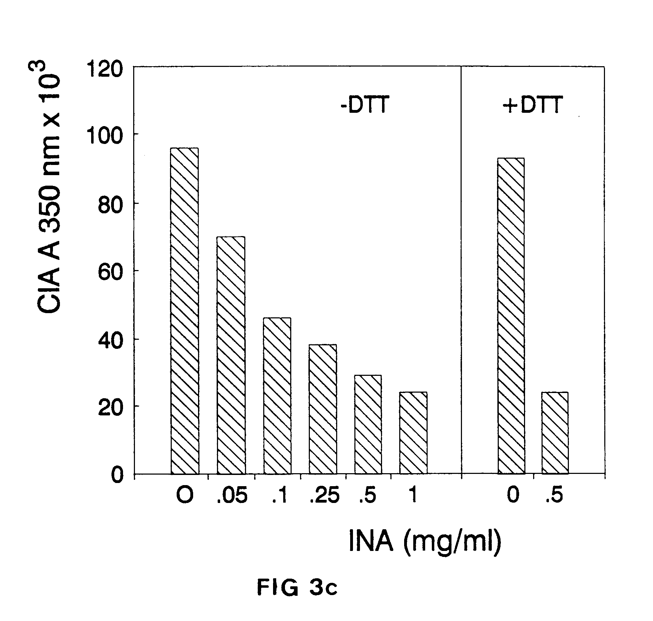

The extinction coefficient of the aFGF has been determined by gas phase acid hydrolysis of aFGF solution of a known optical absorption at around 280 nm, such as described by G. Giménez - Gallego and K.A. Thomas (1987, J. Chromat. 409, 299-304). It was observed that this extinction coefficient was 1.33 (mg/ml)⁻¹ cm ⁻¹, while the same coefficient determined for the aFGF prepared utilizing reverse phase chromatography is 1.09 (mg/ml)⁻¹ cm⁻¹. These results indicate that this last process introduces modifications into the protein structure, and these modifications of the protein are probably those responsible of the poor solubility in aqueous solutions at physiological pH, a problem which is accentuated the closer the solution comes to temperatures proximate to that of warm - blooded animals. The insolubility has been measured by turbidimetry at a wavelength of 350 nm, adding to the cuvette protein up to 13 to 18 ug/ml of protein. Figure 3a shows how the precipitation of the protein, prepared by including a reverse phase chromatography stage, increases as the pH does, while Figure 3b shows how, as the temperature rises, the precipitation of the aFGF increases. As taken up in Figure 3c, this precipitation can be partially avoided, adding heparine to the medium and these insolubility problems disappear in the protein that has been prepared in accordance with the process described above and making the reverse phase chromatography unnecessary.

MITOGENIC ACTIVITY OF THE aFGF

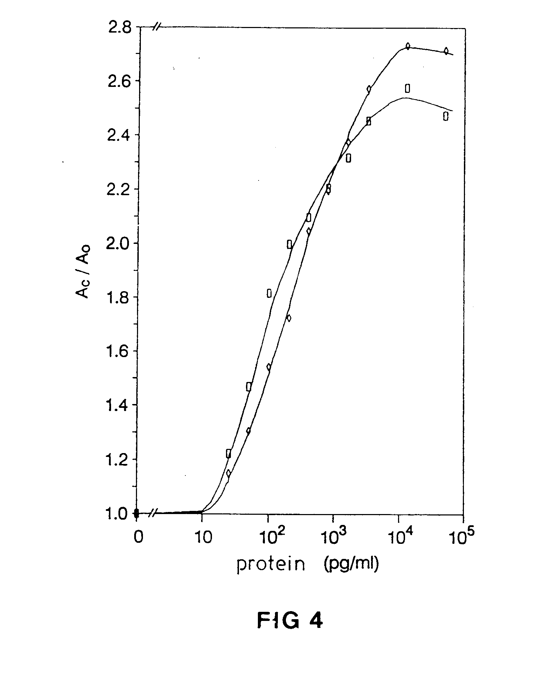

The mitogenic activity of the aFGF purified according to the process described above has been tested in Balb/c3T3 fibroblasts. For the test, fibroblasts, resuspended in 10% bovine foetal serum in Dulbecco modified Eagle medium (DMEM), were seeded at a density of 10600 cells per cm² in plates with 96 wells which have first been covered with fibronectine at 1 ug per cm². After six hours the medium was withdrawn from the wells and 0.1 ml of quiescence medium added (HAM/DMEM medium 1:1, L-glutamine 2mM, SeO₃Na₂ luM, transferrine 5 ug/ml, 2% penicillin/streptomycene) and twenty four hours later increasing quantities of aFGF in 0.01 ml of DMEM were added to which bovine serumalbumin was added up to 1 mg/ml and sodium heparine up to 0.5 mg/ml. After forty eight hours the cells were fixed with 10% formaldehyde in saline phosphate buffer for 30 minutes at room temperature. Then the formaldehyde was removed and the plates dried at 37° C during 15 minutes. Once dried, the fixed cells were stained with 0.55 crystal violet 5% in distilled water for 10 minutes. The excess of colorant was removed by three successive washings with distilled water. The amount of colorant retained by the cells, proportional to the number of cells in the well, was determined by an Elisa plate reader measuring the optical absorption difference between 600 and 450 nm, once the colorant was solubilized adding methanol to the wells. In Figure 4 is represented the quotient between the optical absorption difference of the wells at the various concentrations of aFGF (Ac) and those to which the growth factor was not added (Ao) against the amount of aFGF added. The activity unit of the complete aFGF, prepared as described, expressed as the protein concentration causing a mitogenic activity one half the maximum is 6.63 x $10^{12}$ M. As seen in the same figure, the unit of activity of the shorter forms of the aFGF, prepared avoiding the reverse phase chromatography, coincides with the published values (Linemayer, D.L. et al. 1987 Bio/technology 5, 960-965) and is almost three times less (17.1 x $10^{-12}$ M) than in the case of the complete form.

As mentioned earlier, a further object of the invention is also the use of the fibroblast growth factor as a drug for preventing myocardial ischemia/reperfusion injuries.

In the heart, following an ischemic insult, it is proposed to open the ATP-sensitive potassium channels ($K^+$), since the openers of these channels have been shown to reduce the ischemic/reperfusion damage in isolated rat hearts (Quast, W. and Cook, N.S. 1989, Trends Pharmacol. Sci. 10, 431-435; Grover, G.J., Sleph, P.G. & Dzwonczyk, S. 1990, J. Mol. Cell. Cadiol. 22, -Supplement I- page 6). The ensuing hyperpolarization would prevent calcium ions ($Ca^{2+}$) entry through the voltage operated $Ca^{2+}$ channels thereby reducing $Ca^{2+}$ overload. The outward current induced by the activation of the ATP - sensitive $K^+$ channels should also shorten the plateau of the ventricular action potential originating a reduced contraction. In addition, the outward shunt conductance that results from channel activation will increase the threshold for electrical excitation and so slow pacemaker activity. Hence, the opening of these $K^+$ channels could provide an important safety mechanism in the case of ischemic myocardium (Quast, W. and Cook, N.S. 1989, Trends Pharmacol. Sci. 10, 431-435; Ashcroft F.M. 1988, Ann. Rev. Neurosci. 11, 97-118). It seems obvious that the fibroblast growth factor, both acidic and basic (aFGF, bFGF), should be released at the ischemic tissue, either or both from the injured cells and from the extracellular matrix, or else from one or the other (Baird, A. and Ling, N. 1987, Biochem. Biophys. Res. Commu. 142, 428-435; Kardami, E. and Fandrich, R.R. 1989, J. Cell Biol. 109, 1865-1875). Recently it has been shown that aFGF selctively accumulates in cardiomyocites, at the ischemic collateralized pig myocardium (Bernotat - Danielowski, S., Schott, R.J., Quinkler, W., Sharma, N.S., Kremer, P. and Schaper, W. 1990, J. Mol. Cell Cardiol. 22, -Supplement III- page 29). Since FGF has been shown to induce "in vivo" opening of ATP - sensitive $K^+$ channels (Cuevas, P. et al., unpublished results), it is reasonable to expect that this protein protects myocardium from ischemic/reperfusion injury. This expectation is duly corroborated by the following tests:

Tests

In these tests Wistar rats of either sex are used, with a weight of 200 - 250 g. For at least one week prior to experiments the animals were housed in groups of 2 - 5 on a standard lightdark cycle (light on 6.00 - 18.00 hours), with rat chow pellets and water continously available. The colony room had a temperature of 20 - 22° C and humidity of 45 - 55%. All applicable guidelines for care and use of animals for experimentation were followed. All the experiments were performed in anaesthetized animals (3 i.p. ml per kg weight of a mixture of Ketamine hydrochloride 2.5 mg/ml, atropine 0.1 mg/ml and valium 2 mg/ml). The depth of anaesthesia was ascertained by the lack of corneal reflex.

Surgical process

Under a surgical operating microscope (Zeiss OPMI) a transient myocardial ischemia was performed using techniques described in detail in Fishbein MC, Maclean D, Maroko PR. "Experimental myocardial infarction in the rat. Qualitative and quantitative changes during pathologic evolution". Am. J. Pathol. 1978, 90, 57-70; Selye H., Bajusz E, Grasso S, Mendell P. "Simple techniques for the surgical occlusion of coronary vessels in the rat". Angiology, 1960, 11, 398-407; Fabiani JN, Deloche A, Camilleri JP, Joseph D, Carpentier A, Dubost C. "Protocoles d'étude de l'ischémie aigüe du myocarde chez le rat". Ann. Chir. Thorac. Cardiovasc. 1977, 16, 459-470. For the purpose, by a left lateral thoracotomy the chest was opened, the fifth and sixth ribs were separated and the pericardium was scised. The heart was exteriorized by applying pressure to the lateral wall of the thoracic cage, and the left main coronary artery was then occluded 1 to 2 mm from their origin with a ligature passed through the myocardial tissue around the artery. The heart was then replaced within the thoracic cavity with the ligature ends exteriorized. At this point, any animals exhibiting significant ventricular arrhytmias were discarged from the study. Both ends of the ligature were passed through a small plastic tube which was then pressed on the surface of the heart directly above the left coronary artery. The entire surgical procedure took 1 - 2 minutes. The resulting arterial occlusion was maintained for 10 minutes by clamping the plastic tube and ligature with a small hemostat. The thoracic cage within the pectoral musculature was then sutured and reperfusion was achieved by removing the hemostat in the plastic tube and the ligature. The fact of choosing an occlusion time of 10 minutes was because reperfusion injury has been shown to occur in hearts reperfused after 10 minutes of ischemia (Garlick et al., 1987, Circ. Res. 61, 757-760) and because the release of creatininkinase (CK) into the blood from infarcted heart rises within 8 to 24 hours (Pasternak and Braunwald, 1990, acute myocardial infarction, in: Harrison's Principals of Internal Medicine, 12th edition [E. Braunwald et al., eds.] McGraw & Hill, New York). The electrocardiogram (ECG) was recorded throughout the experimental time course via standard limb leads. Validation of successful coronary occlusion and reperfusion was available from the accompanying electrocardiogram changes in ST - segment elevation and R - wave height. Sham - operated animals underwent the same surgical procedure, with the exception that the suture passed around the coronary artery was not tied. Rats were allowed to recover for 24 hours, during which time food and water were provided "ad libitum". All animals were anaesthetized, as indicated above, 24 hours after the operation and CK activity was measured on left ventricular and interventricular septum homogenates.

Detection of the creatininquinase activity

All the tested animals were reanaesthetized as indicated above, and their hearts were extirpated while still beating. The left ventricular ischemic area and the non - ischemic interventricular septum was collected and stored at -70° C before CK assay. After tissue homogenization, the supernatant was collected and CK activity was determined by spectrophotometric assay according to Miranda (Miranda, A.F., Babiss, L.E. and Fisher, B.P. Methods in Enzymology, 1986. vol 119, 619-634). Protein content was estimated by the Bradford method (Bradford, M., Anal. Biochem. 1976, 72, 248-254).

Statistical analysis

All data was expressed as mean ± standard deviation. Analysis of variance and Students test was used to compare the values among groups. The results are indicated in Table 1.

| | |
|---|---|
| Mean: | Mean of differences in CK activities between the ventriculum and septum of the same heart in the various treatments. |
| Standard Deviation: | Standard deviation of the differences between the CK activities between ventriculum and septum. |
| Standard Deviation (diff): | Standard deviation of the estimated average for the distribution of the differences between the averages of the samples of a single population. |
| [M1-M2]: | Difference in the mean of the samples compared. |
| z: | Differences in standard deviations of M1-M2 with respect to the extimated average distribution between the means of the samples of a single population. |
| p: | Probability of the samples compared to proceed from a single population. |

Tabelle 1

| | Mean | S.D. | S.T.(df) | [M1-M2] | z | p |
|---|---|---|---|---|---|---|
| 1. No infarct | 0,33656 | 0,09092 | | | | |
| 2. Infarct/untreated | -0,65890 | 0,81179 | | | | |
| 3. bFGF (time 10) | 0,10555 | 0,44206 | | | | |
| 4. bFGF (time -5 min) | -0,38700 | 0,41502 | | | | |
| 5. bFGF (time 1 min) | 0,33500 | 0,13305 | | | | |
| 6. aFGF (time 1 min) | 0,37800 | 0,29614 | | | | |
| 7. aFGF non-mitogenic (time 1 min) | 0,45111 | 0,21281 | | | | |
| 8. No infarct/untreated | | | 0,315852 | 0,995456 | 3,151653 | <0,002 |
| 9. no infarct/bFGF (time 10) | | | 0,240371 | 0,231011 | 0,961060 | <0,35 |
| 10. Untreated/bFGF (time 0) | | | 0,343262 | 0,764445 | 2,227002 | <0,03 |
| 11. No infarct/bFGF (time -5 min) | | | 0,235409 | 0,723556 | 3,073612 | <0,0025 |
| 12. Untreated/bFGF (time -5 min) | | | 0,350259 | 0,271900 | 0,776283 | 0,45 |
| 13. No infarct/bFGF (time 1 min) | | | 0,154605 | 0,001556 | 0,010064 | <0,992 |
| 14. Untreated/bFGF (time 1 min) | | | 0,307382 | 0,993900 | 3,233436 | <0,002 |
| 15. No infarct/aFGF (time 1 min) | | | 0,205257 | 0,041444 | 0,201913 | <0,84 |
| 16. Untreated/aFGF (time 1 min) | | | 0,332855 | 1,036900 | 3,115170 | <0,002 |
| 17. No infarct/aFGF n.m. (time 1 min) | | | 0,183707 | 0,114555 | 0,623574 | <0,55 |
| 18. Untreated/aFGF n.m. (time 1 min) | | | 0,334472 | 1,110011 | 3,318696 | <0,00095 |

Conclusions

The preceding tests show that the treatment with the acidic and basic fibroblast growth factor in an animal model of transient myocardial ischemia reduces myocardial CK depletion (measured 24 hours after coronary artery reperfusion).

The date obtained by the variance and Student's test favour therapeutical interest of the acidic and basic fibroblast growth factors with anti - ischemic drug in preventing injuries to the myocardium by ischemic reperfusion.

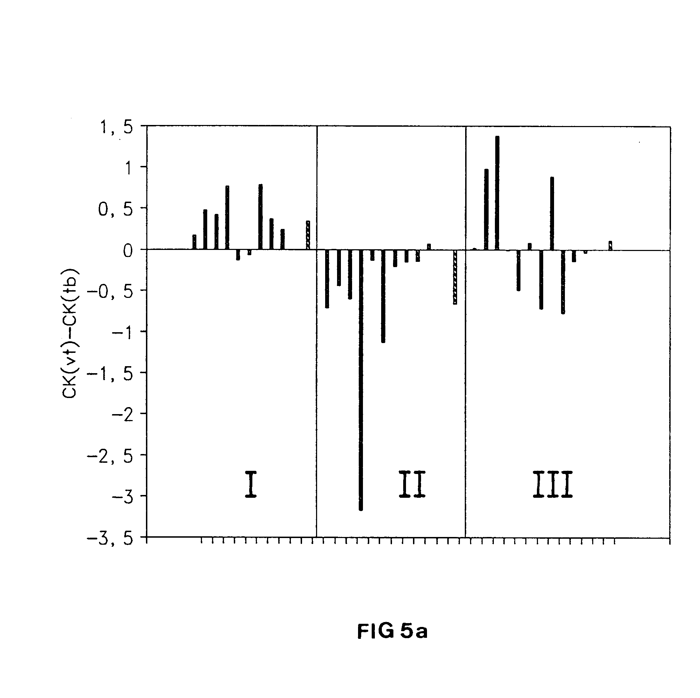

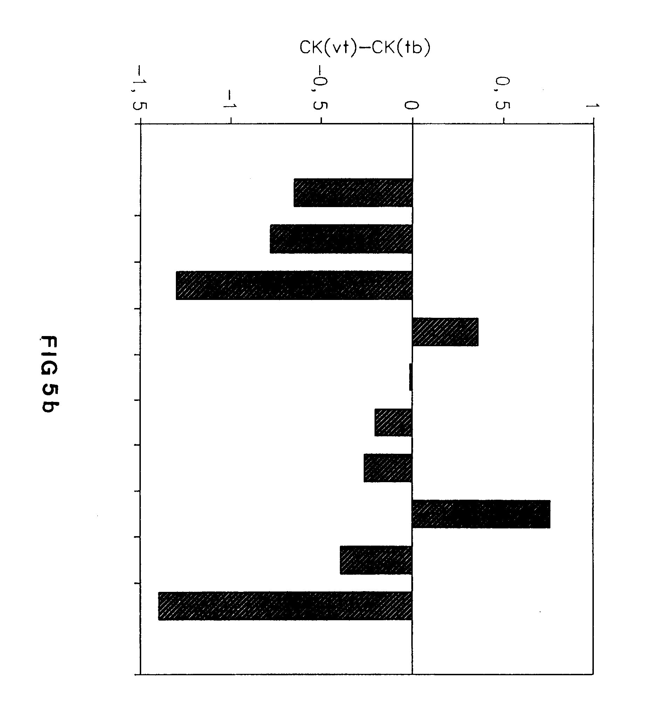

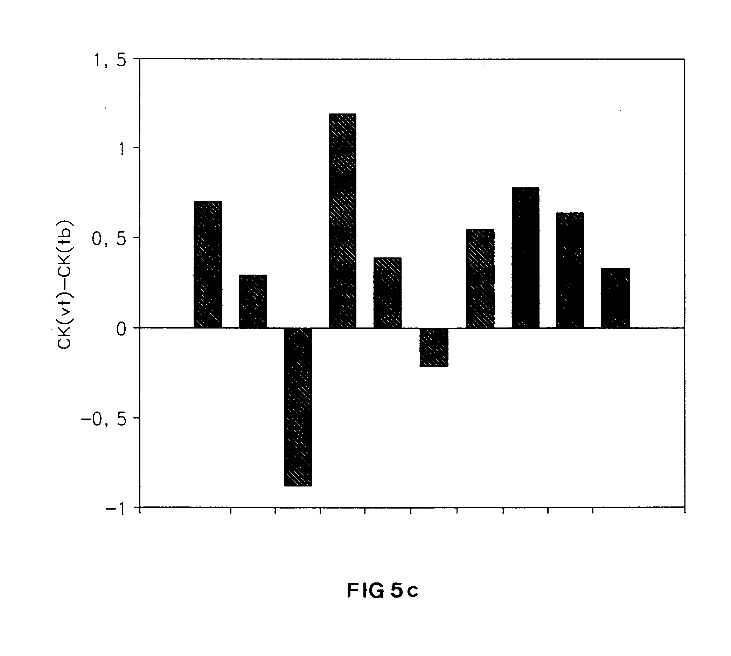

These results are duly corroborated in the graphs represented in Figures 5a to 5e of the drawings.

As can be observed from Figure 5a, therein different states of a heart are represented, not having suffered infarct, having suffered an infarct and into which bFGF has been injected on reperfusion.

Figure 5b represents the administration of bFGF five minutes before reperfusion in a heart which has suffered an infarct.

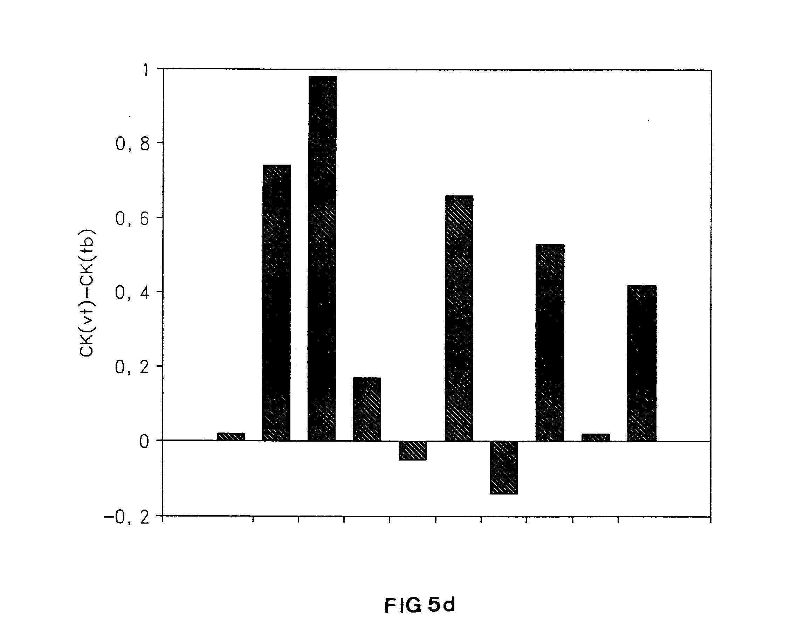

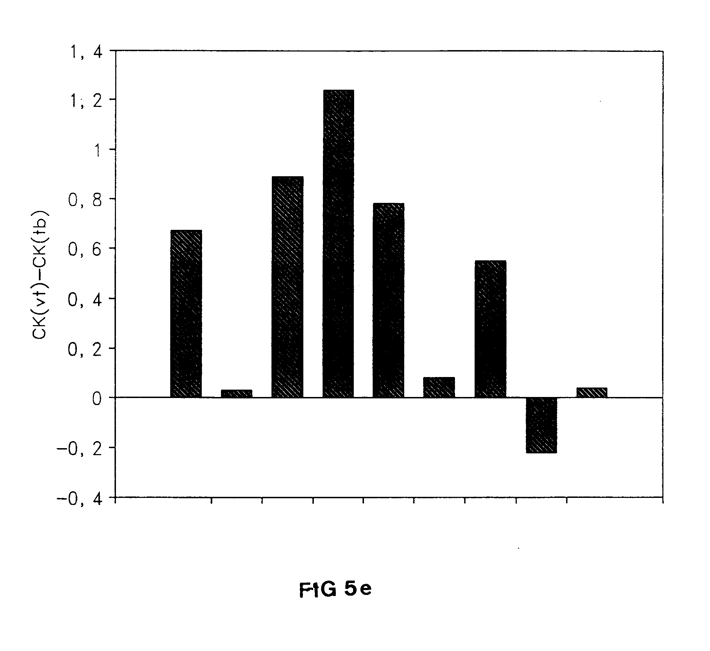

Figures 5c and 5d represent the administration of aFGF and bFGF, respectively, one minute following the reperfusion, and Figure 5e represents the administration of non - mitogenic aFGF one minute following the reperfusion.

As can be observed from the graphs of the mentioned Figures, administration of the fibroblast growth factor, both acidic and basic, and even non - mitogenic, achieves its best results once some time has elapsed (preferably one minute) after the reperfusion, its effect clearly diminishing in the event of being administered before said reperfusion (Figure 5b) and even at the moment of the reperfusion (Figure 5a).

**Claims**

1.  Process for abtaining a new plasmide for producing the acidic fibroblast growth factor (aFGF) in its integral form by genetic engineering techniques, which comprises adding to any codifying nucleotide sequence of the complete aminoacids sequence of aFGF, at the beginning and end, respectively, the necessary sequences for binding the mRNA to the ribosome and the effective termination of the transcription, obtaining an intermediate plasmide; and including the fragment which includes the aFGF gen and the binding sequence to the ribosome and of termination of transcription in a vector.

2.  Process in accordance with Claim 1, characterized in that the aFGF gen, located behind the amino - terminal end of the E. coli lipoprotein, separated from latter by two codons of termination in phase, is controlled by the lipoprotein promoter and by a promoter - operator.

3.    Process for obtaining the acidic fibroblast growth factor in its integral form, characterized in that a microorganism is transformed with the plasmide obtained in accordance with Claims 1 and 2, grown in a suitable culture medium, being isolated starting from supernatant material and purified.

4.    Process according to Claim 3, characterized in that the microorganism used are strains of E. coli.

5.    Full length acidic fibroblast growth factor with greater activity than the other known of less molecular weight, characterized in that it does not have methionine in the amino - terminal end, without post - translational modifications, and soluble in aqueous solutions at physiological pH by purification without reverse phase chromatography, expressable in E. coli.

6.    Use of the fibroblast growth factor or of any member of the family of this factor, for preparing a drug for the treatment of injuries in the myocardium by ischemia/reperfusion.

FIG 1

FIG 2

FIG 3a

FIG 3 b

FIG 3c

**FIG 4**

**FIG 5a**

FIG 5 b

**FIG 5c**

FIG 5d

**FIG 5e**